(19) Europäisches Patentamt
European Patent Office
Office européen des brevets

(11) EP 1 375 649 B1

(12) **EUROPEAN PATENT SPECIFICATION**

(45) Date of publication and mention of the grant of the patent:
**07.03.2012 Bulletin 2012/10**

(51) Int Cl.:
***C12N 9/06*** *(2006.01)* ***C12N 1/21*** *(2006.01)*
***C12P 13/00*** *(2006.01)* ***C07K 14/24*** *(2006.01)*
***C12N 15/53*** *(2006.01)*

(21) Application number: **03013226.0**

(22) Date of filing: **12.06.2003**

(54) **D-amino acid oxidase from Arthrobacter protophormiae**

D-Aminosäure-Oxidase aus Arthrobacter protophormiae

D-aminoacide-oxydase de Arthrobacter protophormiae

(84) Designated Contracting States:
**AT BE BG CH CY CZ DE DK EE ES FI FR GB GR HU IE IT LI LU MC NL PT RO SE SI SK TR**

(30) Priority: **19.06.2002 DE 10227493**

(43) Date of publication of application:
**02.01.2004 Bulletin 2004/01**

(73) Proprietor: **Evonik Degussa GmbH**
**45128 Essen (DE)**

(72) Inventors:
- **Hummel, Werner, Dr.**
  **52445 Titz (DE)**
- **Geueke, Birgit, Dr.**
  **52074 Aachen (DE)**
- **Weckbecker, Andrea**
  **52062 Aachen (DE)**
- **Huthmacher, Klaus, Dr.**
  **63571 Gelnhausen (DE)**
- **Weckbecker, Christoph, Dr.**
  **63584 Gründau-Lieblos (DE)**

(56) References cited:
**EP-A- 0 897 006** **EP-A- 1 205 542**
**WO-A-96/27667** **WO-A1-2004/054614**
**US-A- 5 284 754** **US-B1- 6 187 574**

- **DATABASE EMBL [Online] retrieved from EBI Database accession no. AJ000521 XP002253418**
- **LIN LONG-LIU ET AL: "Expression of Trigonopsis variabilis D-amino acid oxidase gene in Escherichia coli and characterization of its inactive mutants" ENZYME MICROB TECHNOL; ENZYME AND MICROBIAL TECHNOLOGY OCT 2000 ELSEVIER SCIENCE INC, NEW YORK, NY, USA, vol. 27, no. 7, October 2000 (2000-10), pages 482-491, XP002253416**
- **JOB VIVIANA ET AL: "Overexpression of a recombinant wild-type and His-tagged Bacillus subtilis glycine oxidase in Escherichia coli." EUROPEAN JOURNAL OF BIOCHEMISTRY, vol. 269, no. 5, March 2002 (2002-03), pages 1456-1463, XP002253417 ISSN: 0014-2956**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 375 649 B1

SEQUENCE LISTING

<110> Degussa AG

<120> cloning and Expression of D-Amino Acid oxidase from Arthrobacter protophormiae

<130> 020329 BT

<160> 8

<170> PatentIn version 3.1

<210> 1
<211> 981
<212> DNA
<213> Arthrobacter protophormiae

<400> 1

```
atgcccacag caccgttgag aatcaccgtg atcggttccg gcgtcatcgg cctgtccgcg      60
gcccacgagc tggccgccgc cgggcaccag gtcaccgtcg cctacgacca ggagctcgcc     120
gagtgcgtct cctcggtcgc cgccgccatc tggttcccgt accactcgga gaactccccg     180
gccgccgaca agctgctggc ggattcgctg gcccgcttcg agcagctgtc cgagcacccc     240
gaaaccggca tcgacctgcg ccgcggcttg aatgtggacc acctgccggg cgcggaccgc     300
agctggaccc gcatcgtcgc cggcaccgag gaagcctctc ccgcggatct gccggacggc     360
gcccacgcgg gcgtgtgggc gacggtgccg atcattacca tgagcaccta cctgggctgg     420
ctgcgcggcc gggtcgagga gctgggcgcg gacttcgcca agggcacggt cacggatctg     480
gcgcagctta agggcggcgc ggatctggtg gtgctggcag cgggcctgcg cggcggcgag     540
ctgctgggcg acgacgatac cgtctacccg atccgcgggc aggtggtgcg cctggccaac     600
acgaagaacc tgacgcagtg gctgtgcgac gacaactacc cggatggcgt cagctacatc     660
atcccccgcc gcgaggacat catcgtggga ggcaccgaca ccgcgaacga ctggaaccgc     720
gaagtcgaac cgcagacctc catcgacatc ctggaacgcg ctgccaagct ggtgccggag     780
ctggaaggac tggaggtcct ggagcacaag gtgggcctgc gcccggcgcg cgagaccatc     840
cggctggagc acgtcgcggg ccacccgctg ccggtgatcg ccgcctacgg gcacggcggt     900
gccggcgtca cgctgtcctg ggcaccgca cagcgggtcg cagagctggc tgcacaactg     960
gctggcgagc cggccagcta g                                               981
```

<210> 2
<211> 326
<212> PRT
<213> Arthrobacter protophormiae

<400> 2

```
Met Pro Thr Ala Pro Leu Arg Ile Thr Val Ile Gly Ser Gly Val Ile
1               5                   10                  15

Gly Leu Ser Ala Ala His Glu Leu Ala Ala Ala Gly His Gln Val Thr
            20                  25                  30
```

```
Val Ala Tyr Asp Gln Glu Leu Ala Glu Cys Val Ser Ser Val Ala Ala
        35                  40                  45

Ala Ile Trp Phe Pro Tyr His Ser Glu Asn Ser Pro Ala Ala Asp Lys
    50                  55                  60

Leu Leu Ala Asp Ser Leu Ala Arg Phe Glu Gln Leu Ser Glu His Pro
65                  70                  75                  80

Glu Thr Gly Ile Asp Leu Arg Arg Gly Leu Asn Val Asp His Leu Pro
                85                  90                  95

Gly Ala Asp Arg Ser Trp Thr Arg Ile Val Ala Gly Thr Glu Glu Ala
            100                 105                 110

Ser Pro Ala Asp Leu Pro Asp Gly Ala His Ala Gly Val Trp Ala Thr
        115                 120                 125

Val Pro Ile Ile Thr Met Ser Thr Tyr Leu Gly Trp Leu Arg Gly Arg
    130                 135                 140

Val Glu Glu Leu Gly Ala Asp Phe Ala Lys Gly Thr Val Thr Asp Leu
145                 150                 155                 160

Ala Gln Leu Lys Gly Gly Ala Asp Leu Val Val Leu Ala Ala Gly Leu
                165                 170                 175

Arg Gly Gly Glu Leu Leu Gly Asp Asp Asp Thr Val Tyr Pro Ile Arg
            180                 185                 190

Gly Gln Val Val Arg Leu Ala Asn Thr Lys Asn Leu Thr Gln Trp Leu
        195                 200                 205

Cys Asp Asp Asn Tyr Pro Asp Gly Val Ser Tyr Ile Ile Pro Arg Arg
    210                 215                 220

Glu Asp Ile Ile Val Gly Gly Thr Asp Thr Ala Asn Asp Trp Asn Arg
225                 230                 235                 240

Glu Val Glu Pro Gln Thr Ser Ile Asp Ile Leu Glu Arg Ala Ala Lys
                245                 250                 255

Leu Val Pro Glu Leu Glu Gly Leu Glu Val Leu Glu His Lys Val Gly
            260                 265                 270

Leu Arg Pro Ala Arg Glu Thr Ile Arg Leu Glu His Val Ala Gly His
        275                 280                 285

Pro Leu Pro Val Ile Ala Ala Tyr Gly His Gly Gly Ala Gly Val Thr
    290                 295                 300
```

Leu Ser Trp Gly Thr Ala Gln Arg Val Ala Glu Leu Ala Ala Gln Leu
305 310 315 320

Ala Gly Glu Pro Ala Ser
325

<210> 3
<211> 20
<212> PRT
<213> Arthrobacter protophormiae

<400> 3

Pro Thr Ala Pro Leu Arg Ile Thr Val Ile Gly Ser Gly Val Ile Gly
1 5 10 15

Leu Ser Ala Ala
20

<210> 4
<211> 14
<212> PRT
<213> Arthrobacter protophormiae

<400> 4

Leu Val Pro Glu Leu Glu Gly Leu Glu Val Leu Glu His Lys
1 5 10

<210> 5
<211> 27
<212> DNA
<213> Artificial

<220>
<223> Primer Apfor3

<400> 5
gcaccaggtc accgtcgcct acgacca 27

<210> 6
<211> 26
<212> DNA
<213> Artificial

<220>
<223> Primer Aprev3

<400> 6
caccagcttg gcagcgcgtt ccagga 26

<210> 7
<211> 27

<212> DNA
<213> Artificial

<220>
<223> Primer Apfor4

<400> 7
cgccgccgcc atctggttcc cgtacca 27

<210> 8
<211> 30
<212> DNA
<213> Artificial

<220>
<223> Primer Aprev4

<400> 8
ctgcggttcg acttcgcggt tccagtcgtt 30

## Description

The invention relates to the microbial production of D-amino oxidase (D-AAO).

A nucleotide sequence encoding said enzyme has been isolated from Arthrobacter protophormiae and cloned. The coding sequence has been inserted into an expression construct which in turn has been inserted into a vector capable of transforming a microbial expression host. The transformed microbial hosts may be used to economically produce D-AAO. The D-AAO produced via the present invention may be used to oxidize D-amino acids.

D-Amino acid oxidases (D-AAOs; EC 1.4.3.3) catalyse the oxidative deamination of D-amino acids to keto acids, ammonia and hydrogen peroxide being liberated. A cofactor of this reaction is FAD, which is reoxidized by molecular oxygen to form $H_2O_2$.

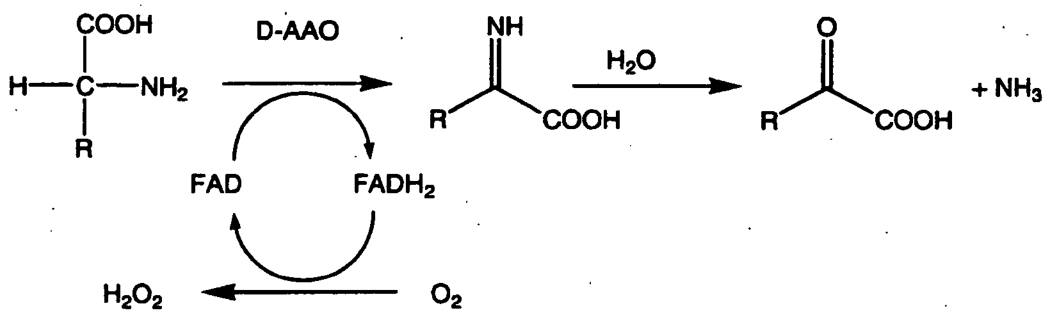


Reaction equation of D-AAO

D-AAO activity was detected in mammalian tissue for the first time in 1935 (Krebs, H.A., Metabolism of amino acids. Deamination of amino acids. Biochem J, 1935. 29: p.77-93). In the following decades D-AAOs were detected from the most diverse mammalian tissues, fish, birds and reptiles (Konno, R., et al., Guinea pig D-amino-acid oxidase cDNA and phylogenetic position. DNA Seq, 1999. 10(2): p. 85-91; Konno, R., Rat D-amino-acid oxidase cDNA: rat D-amino-acid oxidase as an intermediate form between mouse and other mammalian D-amino-acid oxidases. Biochim Biophys Acta, 1998. 1395(2): p. 165-70; Koibuchi, N., et al., Localization of D-amino acid oxidase mRNA in the mouse kidney and the effect of testosterone treatment. Histochem Cell Biol, 1995. 104(5): p. 349-55; Konno, R. and Y. Yasumura, Activity and substrate specificity of D-amino acid oxidase in kidneys of various animals. Zool Mag (Tokyo), 1981. 90: p. 368-73). Microbial D-AAOs have hitherto been characterized only from eukaryotes. Thus, the yeasts Rhodotorula gracilis (Pilone, S.M., A. Vanoni, and P. Casalin, Purification and properties of D-amino acid oxidase, an inducible flavoenzyme from Rhodotorula gracilis. Biochim. Biophys. Acta, 1987. 914: p. 136-142), Trigonopsis variabilis (Berg, C.P. and F.A. Rodden, Purification of D-amino oxidase from Trigonopsis variabilis. Anal. Biochem., 1976. 71(1): p. 214-22) and several Candida species (Gabler, M., M. Hensel, and L. Fischer, Detection and substrate selectivity of new microbial D-amino acid oxidases. Enzyme Microbial Technol, 2000. 27(8): p. 605-611) and the moulds Neurospora crassa (Sikora, L. and G.A. Marzluf, Regulation of L-amino acid oxidase and of D-amino acid oxidase in Neurospora crassa. Mol. Gen. Genet., 1982. 186(1): p. 33-9), Verticillium luteoalbo and various Fusarium species (Gabler, M., M. Hensel, and L. Fischer, Detection and substrate selectivity of new microbial D-amino acid oxidases. Enzyme Microbial Technol, 2000. 27(8): p. 605-611; Isogai, T., et al., Structure and expression of cDNA for D-amino acid oxidase active against cephalosporin C from Fusarium solani. J. Biochem. (Tokyo), 1990. 108(6): p. 1063-9) belong to the D-AAO producers. The D-aspartate and D-glutamate oxidases can be described as a special group, since these enzymes oxidize exclusively acidic D-amino acids and derivatives thereof (Fukunaga, S., et al., Purification and properties of D-glutamte oxidase from Candida boidinii 2201. J. Ferment. Bioengineer., 1998. 85(6): p. 579-583; Wakayama, M., et al., Isolation, enzyme production and characterization of D-aspartate oxidase from Fusarium sacchari var. elongatum Y-105. J. Ferment. Bioengineer., 1994. 5(5): p. 377-379). Bacterial D-AAOs have not yet been characterized.

The most important field of use of D-AAOs is the preparation of 7-aminocephalosporanic acid (7-ACA), a starting substance for semi-synthetic β-lactam antibiotics (Justiz, O.H., R. Fernandez-Lafuente, and J.M. Guisan, One-pot chemoenzymatic synthesis of 3'-functionalized cephalosporines (cefazolin) by three consecutive biotransformations in fully aqueous medium. J. Org. Chem., 1997. 62: p. 9099-9106; Diez, B., et al., Recombinant microorganisms for industrial production of antibiotics. Biotechnol. Bioeng., 1996. 55(1): p. 216-226). In this two-stage process the amino group of the D-aminoadipyl side chain of cephalosporin C is oxidized by the D-AAO and ketoadipyl-7-aminocephalosporanic acid is formed.

A further example of the use of D-AAOs is the preparation of L-6-hydroxynorleucine, an intermediate for the

antihypertensive agent omapatrilat, from the corresponding racemate (Hanson, R.L., et al., Enzymatic synthesis of L-6-hydroxynorleucine. Bioorg. Med. Chem., 1999. 7(10): p. 2247-52; Patel, R.N., Enzymatic synthesis of chiral intermediates for Omapatrilat, an antihypertensive drug. Biomol. Eng., 2001. 17(6): p. 167-82).

A further possible use relates to the preparation of keto acids from D-amino acids by D-AAOs. In this preparation the keto acid as the reaction product must be protected from the $H_2O_2$ formed at the same time in order to prevent decarboxylation. This is achieved by co-immobilization of catalase, both the oxidation of the D-AAO itself and the decarboxylation of the keto acid by $H_2O_2$ being avoided (Buto, S., et al., Evaluation of D-amino acid oxidase from Rhodotorula gracilis for the production of alpha-keto acids: a reactor system. Biotechnol. Bioeng., 1994. 44: p. 1288-1294; Fernandez-Lafuente, R., V. Rodriguez, and J.M. Guisan, The coimmobilization of D-amino acid oxidase and catalase enables the quantitative transformation of D-amino acids (D-phenylalanine) into alpha-keto acids (phenylpyruvic acid). Enzyme Microbial Technol., 1998. 23: p. 28-33). D-AAOs represent a further field of use as signallers in biosensors (Varadi, M., et al., Determination of the ratio of D-and L-amino acids in brewing by an immobilised amino acid oxidase enzyme reactor coupled to amperometric detection. Biosens. Bioelectron., 1999. 14(3): p. 335-40; Sarkar, P., et al., Screen-printed amperometric biosensors for the rapid measurement of L-and D-amino acids. The Analyst, 1999. 124: p. 865-870).

Practically all the known D-AAOs convert only a limited spectrum of amino acids. An up-to-date comparative overview of the substrates which can be converted for six D-AAOs is contained in the publication by Gabler et al. (Gabler, M., M. Hensel, and L. Fischer, Detection and substrate selectivity of new microbial D-amino acid oxidases. Enzyme Microbial Technol, 2000. 27(8): p. 605-611). It shows that all the enzymes listed can convert, for example, basic amino acids to a poor degree or not at all.

The object of the invention is to provide a D-amino acid oxidase (D-AAO) which is selective and also converts basic amino acids.

The invention provides an isolated polynucleotide which encodes a protein comprising the amino acid sequence of SEQ ID NO:2, wherein said protein has D-amino acid oxidase (D-AAO) activity. The polynucleotide is isolated from a strain of Arthrobacter protophromiae.

The invention also provides the above-mentioned polynucleotide, this preferably being a DNA which is capable of replication, comprising:

1) an isolated polynucleotide comprising SEQ ID NO:1 which encode a protein consisting of the amino acid sequence of SEQ ID NO:2;

2) an isolated polynucleotide comprising the complement of SEQ ID NO:1 variants thereof which are results of the degeneracy of the genetic code;

3) an isolated polynucleotide comprising SEQ ID NO:1 or variants thereof which are results of the degeneracy of the genetic code;

4) an isolated polynucleotide which is at least 90 % identical to the polynucleotide of SEQ ID NO:1 and encodes a protein having D-amino oxidase activity;

5) an isolated polynucleotide which hybridizes under stringent conditions to the complement of SEQ ID NO:1, wherein said stringent conditions comprise washing in 0,1XSSC at a temperature from 50 to 68°C, and wherein the polynucleotide encodes a protein having D-amino acid oxidase activity.

The invention also provides polynucleotides, which substantially comprise a polynucleotide sequence, which are obtainable by screening by means of the claimed hybridization of a corresponding gene library of a coryneform bacterium, which comprises the complete gene or parts thereof, with a probe which comprises the sequence of the polynucleotide according to the invention according to SEQ ID No.1 or a fragment thereof, and isolation of the polynucleotide sequence mentioned.

Polynucleotides which comprise the sequences according to the invention are suitable as hybridization probes for RNA, cDNA and DNA, in order to isolate, in the full length, nucleic acids or polynucleotides or genes which code for D-amino acid oxidase or to isolate those nucleic acids or polynucleotides or genes which have a high similarity with the sequence of the dao gene. They are also suitable for incorporation into so-called "arrays", "micro arrays" or "DNA chips" in order to detect and determine the corresponding polynucleotides.

Polynucleotides which comprise the sequences according to the invention are furthermore suitable as primers with the aid of which DNA of genes which code for D-amino acid oxidase can be prepared by the polymerase chain reaction (PCR).

Such oligonucleotides which serve as probes or primers comprise at least 25, 26, 27, 28, 29 or 30, preferably at least 20, 21, 22, 23 or 24, very particularly preferably at least 15, 16, 17, 18 or 19 successive nucleotides. Oligonucleotides with a length of at least 31, 32, 33, 34, 35, 36, 37, 38, 39 or 40 or at least 41, 42, 43, 44, 45, 46, 47, 48, 49 or 50 nucleotides are also suitable. Oligonucleotides with a length of at least 100, 150, 200, 250 or 300 nucleotides are optionally also suitable.

"Isolated" means separated out of its natural environment.

"Polynucleotide" in general relates to polyribonucleotides and polydeoxyribonucleotides, it being possible for these to be non-modified RNA or DNA or modified RNA or DNA.

The polynucleotides according to the invention include a polynucleotide according to SEQ ID No. 1 and also those which are at least 90% and very particularly preferably at least 91%, 93%, 95%, 97% or 99% identical to the polynucleotide according to SEQ ID No. 1.

"Polypeptides" are understood as meaning peptides or proteins which comprise two or more amino acids bonded via peptide bonds.

The polypeptides according to the invention include a polypeptide according to SEQ ID No. 2, in particular those with the biological activity of D-amino acid oxidase, and also those which are at least 90% and very particularly preferably at least 91%, 93%, 95%, 97% or 99% identical to the polypeptide according to SEQ ID No. 2 and have the activity mentioned.

The new dao gene from Arthrobacter protophormiae which codes for the enzyme D-amino acid oxidase (EC 1.4.3.3) has been isolated.

One method to isolate the dao gene of Arthrobacter is to first set up a gene library of this microorganism in Escherichia coli (E. coli). The setting up of gene libraries is described in generally known textbooks and handbooks. The textbook by Winnacker: Gene und Klone, Eine Einführung in die Gentechnologie (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up λ vectors by Kohara et al. (Cell 50, 495 - 508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575).

Börmann et al. (Molecular Microbiology 6(3), 317-326)) (1992)) in turn describe a gene library of C. glutamicum ATCC13032 using the cosmid pHC79 (Hohn and Collins, 1980, Gene 11, 291-298).

To prepare a gene library of C. glutamicum in E. coli it is also possible to use plasmids such as pBR322 (Bolivar, 1979, Life Sciences, 25, 807-818) or pUC9 (Vieira et al., 1982, Gene, 19:259-268). Suitable hosts are, in particular, those E. coli strains which are restriction- and recombination-defective, such as, for example, the strain DH5αmcr, which has been described by Grant et al. (Proceedings of the National Academy of Sciences USA, 87 (1990) 4645-4649). The long DNA fragments cloned with the aid of cosmids or other λ vectors can then in turn be subcloned and subsequently sequenced in the usual vectors which are suitable for DNA sequencing, such as is described e. g. by Sanger et al. (Proceedings of the National Academy of Sciences of the United States of America, 74:5463-5467, 1977).

Also described is a vector containing an expression construct which further contains at least one copy of at least one, preferably homologous DNA sequence encoding D-amino acid oxidase operably linked to an appropriate regulatory region capable of directing the high level expression of peptides or proteins having D-amino acid oxidase activity in a suitable expression host (expression construct).

The expression construct provided by the present invention may be inserted into a vector, preferably a plasmid, which is capable of transforming a microbial host cell and integrating into the genome.

It is a further object of the present invention to provide a recombinant host which has been transformed by a vector as described in the preceding paragraph.

Vector DNA can be introduced into prokaryotic or eukaryotic cells via conventional transformation or transfection techniques. As used herein, the terms "transformation" and "transfection", "conjugation" and "transduction" are intended to refer to a variety of art-recognized techniques for introducing foreign nucleic acid (e. g., linear DNA or RNA (e. g., a linearized vector or a gene construct alone without a vector) or a nucleic acid in the form of a vector (e. g., a plasmid, phage, phasmid, phagemid, transposon or other DNA) into a host cell, including calcium phosphate or calcium chloride co-precipitation, DEAE-dextran-mediated transfection, lipofection, natural competence, chemical-mediated transfer, or electroporation. Suitable methods for transforming or transfecting host cells can be found in Sambrook, et al. (Molekular Cloning: A Laboratory Manual. 2nd, ed., Cold Spring Harbor Laboratory, Cold Spring Harbor Laboratory Press, Cold Spring Harbor, NY, 1989).

Coding DNA sequences which result from SEQ ID No. 1 by the degeneracy of the genetic code are also a constituent of the invention. In the same way, DNA sequences which hybridize under claimed conditions with SEQ ID No. 1 are a constituent of the invention. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. Such mutations are also called, inter alia, neutral substitutions. It is furthermore known that changes on the N and/or C terminus of a protein cannot

substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the invention

In the same way, DNA sequences which hybridize under claimed conditions with SEQ ID No. 1 are a constituent of the invention. Finally, DNA sequences which are prepared by the polymerase chain reaction (PCR) using primers which result from SEQ ID No. 1 are a constituent of the invention. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology 41: 255-260 (1991)). The hybridization takes place under stringent conditions, that is to say only hybrids in which the probe and target sequence, i. e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50°C - 68°C, for example, is employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50°C - 68°C being established. It is claimed to lower the salt concentration to 0.1x SSC. Polynucleotide fragments which are, for example, at least 70% or at least 80% or at least 90% to 95% or at least 96% to 99% identical to the sequence of the probe employed can be isolated by increasing the hybridization temperature stepwise from 50°C to 68°C in steps of approx. 1 - 2°C. It is also possible to isolate polynucleotide fragments which are completely identical to the sequence of the probe employed. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait: Oligonucleotide Synthesis: A Practical Approach (IRL Press, Oxford, UK, 1984) and in Newton and Graham: PCR (Spektrum Akademischer Verlag, Heidelberg, Germany, 1994).

It has been found that different microorganisms produce D-AAO in an improved manner after enhancement of the dao gene of Arthrobacter protophormiae.

The transformed microorganisms which can be used as hosts for said dao gene can prepare D-AAO from glucose, sucrose, lactose, fructose, maltose, molasses, starch, cellulose or from glycerol and ethanol. These are fungi, yeasts or, in particular, Gram-positive bacteria or Gram-negative bacteria, such as e. g. those of the Enterobacteriaceae. Of the family of the Enterobacteriaceae, the genus Escherichia with the species Escherichia coli may be mentioned in particular.

The term "enhancement" in this connection describes the increase in the intracellular activity of one or more enzymes (proteins) in a microorganism which are coded by the corresponding DNA, for example by increasing the number of copies of the gene or allele, using a potent promoter or using a gene or allele which codes for a corresponding enzyme having a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The invention also provides the transformed bacteria which comprise an increased number of copies of the dao gene, wherein said dao gene comprises the polynucleotide sequence of SEQ ID NO:1.

The D-amino acid oxidase of Arthrobacter is produced by a process comprising

a) culturing a suitable microorganism, especially of the family Enterobacteriaceae comprising an overexpressed dao in a medium suitable for expression of the dao gene; wherein said dao gene comprises the nucleotide sequence of SEQ ID NO:1 or any of the claims 1 to 6 and 10 to 12,

b) enriching the D-amino acid oxidase produced in the cells of the bacteria, and optionally

c) isolating said D-amino acid oxidase.

D-amino acid oxidase produced via the present invention may be applied to variety of processes which require the conversion of D-amino acids.

The production according to the present invention will reduce production costs of microbial D-amino acid oxidase in order to allow its economical application.

The D content of enantiomer mixtures is deaminated and converted into the corresponding keto acid.

The L content of the amino acid employed remains in a high enantiomer purity. The separation of keto acid and L-amino acids can be carried out by processes known to the expert, for example by selective precipitation in acid aqueous solution or by ion exchange chromatography. It is advantageous that the enzyme according to the invention has a broad substrate spectrum, so that many naturally occurring and non-naturally occurring amino acids can be converted. The equilibrium of the reaction lies far on the right-hand side of the reaction equation, so that the reaction proceeds quantitatively. The enzyme preferably contains FAD as a cofactor. An external cofactor regeneration is in general not necessary, since the oxygen present oxidizes and therefore regenerates the FAD spontaneously.

The $H_2O_2$ formed in the reaction under consideration can be destroyed in a manner known to the expert, e.g. with catalase.

The process according to the invention is preferably carried out in an enzyme membrane reactor (DE 199 10 691).

The enzymes mentioned can be used in the free form as homogeneously purified compounds or as enzymes prepared by a recombinant method. The enzymes can furthermore also be employed as a constituent of an intact guest organism or in combination with the broken-down cell mass of the host organism, which has been purified to any desired extent. The use of the enzymes in immobilized form is also possible (Bhavender P. Sharma, Lorraine F. Bailey and Ralph A. Messing, "Immobilisierte Biomaterialien - Techniken und Anwendungen", Angew. Chem. 1982, 94, 836-852). The immobilization is advantageously carried out by lyophilization (Dordick et al. J. Am. Chem. Soc. 194, 116, 5009-5010; Okahata et al. Tetrahedron Lett. 1997, 38, 1971-1974; Adlercreutz et al. Biocatalysis 1992, 6, 291-305). Lyophilization in the presence of surface-active substances, such as Aerosol OT or polyvinylpyrrolidone or polyethylene glycol (PEG) or Brij 52 (diethylene glycol mono-cetyl ether) is very particularly preferred (Goto et al. Biotechnol. Techniques 1997, 11, 375-378). Use as CLECs is also conceivable (St Clair et al. Angew. Chem. Int Ed Engl. 2000 Jan., 39(2), 380-383).

The term enantiomerically concentrated describes the presence of one optical antipode as a mixture with the other in >50 mol%.

Amino acid in the context of the invention is understood as meaning a naturally occurring or non-naturally occurring $\alpha$- or $\beta$-amino acid, i.e. the radical on the $\alpha$- or $\beta$-C atom of the $\alpha$- or $\beta$-amino acid is derived from a naturally occurring amino acid, such as is described in Beyer-Walter, Lehrbuch der organischen Chemie [Textbook of Organic Chemistry], S. Hirzel Verlag Stuttgart, 22nd edition, 1991, p. 822 et seq., or moreover also from corresponding non-naturally occurring amino acids, which are described e.g. in DE 199 03 268.8.

The purification of racemates chosen from the group consisting of the following amino acids is particularly preferred: methionine, lysine, arginine, phenylalanine, ornithine, leucine, and histidine, in particular methionine and lysine.

The enzyme according to the invention has an absolute enantiomer selectivity and proves to be particularly suitable for conversion of basic D-amino acids.

These include, in particular:

D-lysine, D-arginine and D-ornithine

After the high cell density fermentation of *E. coli* BL21 (DE3) carrying pEID the D-AAO activity reached 153,000 U $liter^{-1}$ fermentation broth. This value is far higher than all values which were published for the expression of recombinant and wild type D-AAOs. Also the specific activity in the crude extracts of recombinant cells (15-30 U $mg^{-1}$) exceeds the published data for all known D-AAOs noticeably (Yu, J., D.-Y., Y.-J. Zhang, S. Yang, R. Li, and Z.-Y. Yuan. 2002, High expression of Trigonopsis variabilis D-amino oxidase in Pichia pastoris. J. Mol. Catal. B: Enzymatic 18:291-7).

The invention is illustrated in more detail in the following examples:

Example 1:

Isolation and characterization of Arthrobacter protophormiae D-217

To isolate D-AAO-forming microorganisms, soil samples were plated out on a nutrient medium with which coryneform bacteria can be concentrated: Na propionate 0.20 g; $KNO_3$ 0.01 g; $KH_2PO_4$ 0.10 g; NaCl 0.25 g; $MgSO_4$-7 $H_2O$ 0.10 g; $CaCO_3$ 0.02 g; trace elements, vitamin solution; cycloheximide 0.05 g; if appropriate agar 20.00 g; final pH 7.0. Microorganisms which grew on this medium were then transinoculated on to a complex medium (DSM 65) with the following composition (per 1 1): glucose 4.00 g; yeast extract 4.00 g; malt extract 10.00 g; agar 20 g; final pH 7.2. The strains were then multiplied in liquid medium (DSM 65), harvested by centrifugation, broken down, and the cell-free supernatant was obtained by centrifugation (= crude extract). This crude extract was then employed as an enzyme

source for the photometric test for D-AAO activity. The test batch contained: 20 μl amino acid soln. (100 mM); 80 μl TEA/o-dianisidine buffer; 10 μl peroxidase soln. (1,000 U/ml); 70 μl $H_2O$; 20 μl crude extract. The measurement was carried out at 436 nm; 1 U here corresponds to an amount of enzyme which converts 1 μmol of amino acid or 1 μmol of o-dianisidine per min (extinction coefficient ε for o-dianisidine = 8.1 $mM^{-1}$ $cm^{-1}$).
Screening gave a bacterium with an intracellular D-AAO activity. The identification showed that this bacterium is a strain of Arthrobacter protophormiae. The strain has the following properties: It is strictly aerobic and Gram-positive, a catalase-positive rod-shaped organism which shows no formation of acid or gas from glucose. Neither a type of mobility nor the presence of spores is to be detected. The final assignment was rendered possible by sequencing of the 16S rDNA.

The strain Arthrobacter protophormiae ID99-1256 was deposited at the DSM under number 15035 on 04.06.2002 at the Sammlung von Mikroorganismen und Zellkulturen GmbH in Braunschweig in accordance with the terms of the Budapest Treaty.

Example 2

High cell density fermentation of E. coli BL21 (DE3) carrying pEID

A fed-batch fermentation was carried out using the following medium for the batch (per liter): 0.2 g $NH_4Cl$, 2 g $(NH_4)SO_4$, 13 g $KH_2PO_4$, 10 g $K_2HPO_4$, 6 g $NaH_2PO_a \cdot H_2O$ , 3 g yeast extract, 2 g glucose, 0.1 g ampicillin, 9.3 mg $Na_2EDTA \cdot 2H_2O$, 0.1 g thiamine, 0.3 ml antifoam, 5 ml vitamin and 4 ml trace element solution. The feed medium consisted of 0.2 g $NH_4Cl$, 2 g $(NH_4)SO_4$, 13 g $KH_2PO_4$, 10 g $K_2HPO_4$, 6 g $NaH_2PO_4$, 21 g yeast extract, 600 g glucose, 10 g $MgSO_4 \cdot 7H_2O$, 0.4 ml antifoam, 5 ml vitamin and 2 ml trace element solution per liter. The vitamin solution contained (per liter): 0.1 g riboflavin, 10 g thiamine/HCl, 0.5 g nicotinic acid, 0.5 g pyridoxine/HCl, 0.5 g Ca-panthotenate, 1 mg biotin, 2 mg folic acid and 10 mg cyanocobalamin. The trace element solution container (per liter) : 10 g $CaCl_2 \cdot H_2O$, 0.5 g $ZnSO_4$·7H2O, 0.25 g $CuCl_2 \cdot 2H_2O$, 2.5 g $MnSO_4 \cdot H_2O$, 1.75 g $CoCl_2 \cdot 6H_2O$, 0.125 g $H_3BO_3$, 2.5 g $AlCl_3$-$6H_2O$, 0.5 g $Na_2MoO_4 \cdot 2H_2O$, 10 g $FeSO_4 \cdot 7H_2O$.

The first preculture of E. coli BL21 (DE3) containing pEID was inoculated in 5 ml $LB_{amp}$ medium from agar plates and cultivated for 14 h at 37°C. These cells were used for the inoculation of the second preculture consisting of 100 ml $LB_{amp}$ in a 500 ml Erlenmeyer flask. After 8 hours of incubation at 37°C it was used to inoculate the fermenter. The fermentation was carried out in a Techfors fermenter using the IRIS software (Infors, Bottmingen, Switzerland). The batch volume was 10 liter and the feed volume 5 liter. For the fed-batch fermentation an exponential feeding strategy according to Korz et al. was slightly modified and applied according to the following equation (Korz, D. J., U. Rinas, K. Hellmuth, E. A. Sanders, and W. D. Deckwer. 1995. Simple fed-batch technique for high cell density cultivation of Escherichia coli. J. Biotechnol. 39:59-65).

$$m_S(t) = \left( \frac{\mu_{set}}{y_{X/S}} + m \right) \cdot V(t_0) \cdot X(t_0) \cdot e^{\mu_{set}(t-t_0)}$$

$m_s$ is the mass flow of substrate (g $h^{-1}$) , $y_{x/s}$ is the biomass/substrate yield coefficient (g $g^{-1}$), $m$ the specific maintenance coefficient (g $g^{-1}$ $h^{-1}$) , $X$ the biomass concentration (g $liter^{-1}$) and V the cultivation volume (liter). The constant specific growth rate $\mu_{set}$ was set to 0.12 $h^{-1}$ to avoid accumulation of acetate. The cells were induced with 5 mM IPTG after 28 h of cultivation. The $pO_2$ was adjusted to 30% by adapting the stirrer speed, the pressure and the aeration rate.

The pH was maintained at 7 by adding NH4OH and the temperature was kept at 30°C. The cells were harvested with a separator KA06 (Westfalia, Oelde, Germany) after a fermentation time of 39 h.

After the high cell density fermentation of E. coli BL21(DE3) carrying pEID 1.9 kg of cells (wet weight) were harvested which had an activity of 1200 U $g^{-1}$ cells (wet weight). The specific activity was 15 U $mg^{-1}$. We obtained 2.29 mill. Units D-AAO from one 15 liter-fermentation.

Example 3:

3 a) Purification of the D-AAO from Arthrobacter protophormiae D-217

It was possible to obtain homogeneous D-AAO by a three-stage purification. The purification protocol is summarized in table 1. After two chromatography purification steps, the active fractions were combined, dialysed and purified further by means of preparative, native gel electrophoresis. The yield was 8.5% and the D-AAO was concentrated 62-fold.

3 b) Purification of recombinant D-AAO

The recombinant D-AAO was isolated to homogeneity using a two-step procedure (TABLE 1). E. coli BL21 (DE3) carrying pApdao was disrupted by ultrasonification. The cell debris was removed by centrifugation. The cleared supernatant was loaded onto a Q-sepharose FF column (h 10 cm x Ø 2.6 cm) (Amersham Bioscience), which was equilibrated with 10 mM potassium phosphate buffer pH 7.0. The flow rate was 2 ml/min. The column was washed with 10 mM potassium phosphate buffer pH 7.0 containing 0.2 M NaCl and the D-AAO was eluted by increasing the salt concentration up to 0.35 M. Active fractions were pooled and loaded onto a column, which was packed with ceramic MacroPrep hydroxyapatite, type I (h 8.3 cm x Ø 1.6 cm) (Bio-Rad). The column was equilibrated with 10 mM potassium phosphate buffer pH 6.8 containing 150 mM NaCl and the flow rate was 2 ml/min. The D-AAO was detected in the flow-through.

The specific activity of 30 U/mg in the crude extract could be increased six-fold to 180 U/mg after the second chromatography. We obtained 1434 U D-AAO from 3 g of cells (wet weight), which corresponds to 56% of the activity in the crude extract. The purification process was monitored by SDS/PAGE (Abb./Fig. 1). TABLE 1: Purification scheme of D-AAO from E. coli BL21(DE3) (3 g cells, wet weight). Activity assay: 0.2 M TEA/HCl, 10 mM D-Met, 100 U/ml peroxidase, 0.2 mg/ml o-dianisidine, D-AAO, pH 7.6, 30°C, 436 nm.

Table 1

| | **Total activity [U]** | **Total protein [mg]** | **Volume [ml]** | **Specific activity [U/mg]** | **Purification[-fold]** | **Yield [%]** |
|---|---|---|---|---|---|---|
| Crude extract | 2556 | 85 | 10 | 30 | 1.0 | 100 |
| Q-Sepharose | 1909 | 24 | 9.7 | 80 | 2.7 | 75 |
| Hydroxyapatite | 1434 | 8 | 12. | 180 | 6.0 | 56 |

Example 4:

Biochemical characterization

A) Optimum pH

The influence of the pH on the activity of the D-AAO with respect to the substrates L-lysine, L-phenylalanine and L-methionine was investigated in three different buffers (citric acid-sodium phosphate pH 4-7, TEA/HCl pH 7.0-8.5, glycine/NaOH pH 8.5-11). The optimum pH for all three substrates was in the weakly basic range between pH 7.5-8.5 (fig. 2). The buffers employed did not substantially influence the enzyme activity.

B) Substrate spectrum

A partial substrate spectrum was recorded for the D-AAO (table 2). In addition to D-methionine and D-lysine as the best substrates, of the hydrophobic D-amino acids D-leucine, D-phenylalanine and D-phenylalanine derivatives were converted.

TABLE 2: Substrate specificity of the recombinant D-AAO. The activities were measured with a final substrate concentration of 10 mM. In case DL-amino acids were used, the concentration was increased to 20 mM. * 300 μl of the supernatant of a saturated substrate solution were used in 1 ml assay volume for amino acid with low solubilities.

Table 2

| **Substrate** | Relative activity [%] |
|---|---|
| D-Methionine | 100 |
| D-Lysine | 99 |
| 4-Fluoro-DL-phenylalanine | 81 |
| D-Arginine | 80 |
| D-Phenylalanine | 54 |
| DL-Homophenylalanine# | 31 |

(continued)

| Substrate | Relative activity [%] |
|---|---|
| D-Ornithine | 15 |
| D-Leucine | 12 |
| D-Histidine | 5.9 |
| D-Tyrosine# | 4.7 |
| N-Methyl-DL-leucine | 2.1 |
| D-allo-Isoleucine | 1.0 |
| D-Valine | 0.35 |
| O-Methyl-DL-serine | 0.33 |
| D-Alanine | 0.26 |
| D-Proline | 0.25 |
| DL-tert-Leucine | 0.12 |

The stereoselectivity of the D-AAO was tested with the amino acids L-leucine, L-methionine and L-lysine, none of these compounds was converted, so that the D-AAO from Arthrobacter protophormiae can be described as highly stereoselective for D-amino acids.

The kinetic constants for the substrates D-methionine and D-phenylalanine were determined with partly purified D-AAO. D-Methionine, with a $K_M$ value of 0.90 mM, had a significantly higher affinity for the D-AAO than D-phenylalanine, the $K_M$ value of which was 16 mM. A substrate excess inhibition was moreover observed for D-phenylalanine ($K_I$ = 55 mM).

Example 5: Protein chemistry characterization

A) Molecular mass and pI

A molecular mass for a monomer of the D-AAO of 40 kDA was determined by means of SDS gel electrophoresis. The native molecular mass was determined to be 69 kDa after gel filtration. The calculated molecular mass of the monomer was 34.6 kDa without and 35.4 kDa with FAD. These values show that the enzyme has a dimeric structure. Isoelectric focussing was performed using precast Serva polyacrylamide gels with a narrow pH range (pH 4-6). The experimentally determined pI was 4.4.

B) Gene and protein sequence data of the D-AAO

The first 20 amino acids of the protein were determined by automated Edman degradation of the N-terminus of the purified D-AAO transferred to a PVDF membrane.

**N-terminus:** PTAPLRITVIGSGVIGLSAA **SEQ ID NO:3**

corresponding to Pro Thr Ala Pro Leu Arg Ile Thr Val Ile Gly Ser Gly Val Ile Gly Leu Ser Ala Ala

A further internal sequence was identified after proteolytic cleavage of the protein and separation of the peptides:

**Internal sequence 1:** (K)LVPELEGLEVLEHK **SEQ ID NO:4**

**(Lys) Leu Val Pro Glu Leu Glu Gly Leu Glu Val Leu Glu His Lys**

Primer were constructed based on the N-terminal sequence information SEQ ID NO:3 and the internal sequence information SEQ ID NO:4. 75% of the gene sequence were amplified using this primer pair. The complete dao gene sequence was isolated with the Universal GenomeWalker™ Kit (BD Clontech). Genomic DNA was cut with six restriction endonucleases NruI, EcoRV, PvuII, StuI, NaeI and FspI (BD Clontech or New England Biolabs). The GenomeWalker adaptors were ligated to the genomic DNA fragments and these libraries were used as templates in PCR reactions. Four primers were designed for the nested PCRs: Apfor3 SEQ ID NO:5- gcaccaggtcaccgtcgcctacgacca, Aprev3 SEQ ID NO: 6 caccagcttggcagcgcgttccagga, Apfor4 SEQ ID NO:7 cgccgccgccatctggttcccgtacca, Aprev4 SEQ ID NO: 8 ctgcggttcgacttcgcggttccagtcgtt. The amplification procedures consisted of 3 min at 94°C followed by 4 cycles of 1 min at 94°C, 30 s at 65°C, 3 min at 72°C, 30 cycles of 1 min at 94°C, 30 sec at 62°C and 3 min at 72°C. The reaction was stopped after a final incubation step for 10 min at 72°C. The PCR products were analysed on agarose gel and used for direct sequencing. The gene sequence and the deduced protein sequence are documented in the appendix.

Example 6: Cloning and expression

The restriction sites NdeI and BamHI were attached to the dao gene by PCR. The PCR product was digested and ligated into pET21a yielding pEID and the DNA was transformed into E. coli XL1 blue and BL21(DE3). E. coli BL21 (DE3) carrying the plasmid pE1D was cultivated aerobically in 100 ml-shaking flasks containing 20 ml Luria-Bertani medium and 100 μg/ml ampicillin. The cells were incubated at 37°C and 200 rpm and induced with 100 μM IPTG when they reached an optical density of $OD_{550}$ ~0.5. After induction the temperature was reduced to 30°C and the cells were incubated for 4 more hours. The specific D-AAO activity in the crude extract reached 30 U/mg.
The plasmid pET21a is available from Novagen, Cat. No. 69740-3.
A figure of plasmid pE1D is shown in Figure 3.

Brief description of the figures

Figure 1

SDS/PAGE of D-AAO containing samples at different stages of purification from E. coli BL21(DE3), corresponding to Table 1. Lane M, marker; lane 1, crude cell extract; lane 2, pool after anion exchange chromatography; lane 3, pool after hydroxyapatite chromatography.

Figure 2

Effect of the pH on the D-AAO activity:

Figure 2a: D-Methionine, Figure 2b: D-Lysine, Figure 2c: D-Phenylalanine.
The enzyme activity was measured with three different substrates in the following 80 mM buffers: ♦ citric acid-sodium phosphate, pH 5-7; ■ TEA/HC1, pH 7-8.5; A glycine/NaOH, pH 8.5-11. Each D-amino acid tested was employed in a final concentration of 10 mM.

Figure 3

Plasmid pE1D

Figure 4

Temperature stability of homogenous D-AAO. The enzyme was incubated at four different temperatures (A 20°C; * 30°C; X 40°C; ♦ 50°C). Residual activity was measured under standard assay conditions.

## Claims

1. An isolated polynucleotide which encodes a protein comprising the amino acid sequence of SEQ ID NO:2, wherein said protein has D-amino acid oxidase activity.

2. The isolated polynucleotide of claim 1, wherein said polynucleotide is isolated from Arthrobacter protophormiae.

3. The isolated polynucleotide of claim 1 or 2 according to SEQ ID NO:1 or variants thereof which are results of the degeneracy of the genetic code.

4. The isolated polynucleotide of claims 1 or 2 consisting of SEQ ID NO:1 or fragments thereof which encode a protein consisting of the amino acid sequence of SEQ ID NO:2.

5. The isolated polynucleotide of claims 1 or 2 comprising the complement of SEQ ID NO:1.

6. The isolated polynucleotide of claims 1 or 2, wherein said nucleotide sequence is at least 90 % identical to the polynucleotide of SEQ ID NO:1.

7. A protein comprising the amino acid sequence of SEQ ID NO:2, and wherein said protein has D-amino acid oxidase activity.

8. An isolated polynucleotide consisting of at least 15 consecutive nucleotides selected from one of SEQ ID NO:1 or a complement of SEQ ID NO:1having the function of a probe in a PCR-reaction that is used to isolate or identify a polynucleotide encoding a protein comprising the amino acid sequence of SEQ ID NO: 2.

9. The isolated polynucleotide of claim 1 which hybridizes under stringent conditions to the complement of SEQ ID NO:1; wherein said stringent conditions comprise washing in 0,1XSSC at a temperature from 50 to 68°C, and wherein the polynucleotide encodes a protein having D-amino acid oxidase activity.

10. An expression construct wherein a DNA sequence of any one of claim 1 to 6 and 9, is operably linked to a regulatory region capable of directing the expression of the D-amino acid oxidase in a suitable expression host.

11. A vector capable of transforming a host cell wherein the vector contains an expression construct according to claim 10.

12. The vector of claim 11, further characterizes in that it is a plasmid.

13. A transformed host cell comprising a vector of claim 10 or 11.

**14.** The transformed host cell of claim 13 which is an bacterium of the Enterobacteriaceae family.

**15.** The transformed host cell of claim 13, which is an bacterium of the genus Escherichia.

**16.** An isolated polypeptide comprising an amino acid sequence that is at least 90 % identical to that of SEQ ID NO:2 and has D-amino acid oxidase activity.

**17.** A process for the production of a D-amino acid oxidase comprising

a) culturing a recombinant microorganism comprising an overexpressed dao gene under conditions conductive for the production of the dao gene product; wherein said dao gene comprises the nucleotide sequence of SEQ ID NO:1 or any of the claims 1 to 6 and 10 to 12 and
b) enriching the D-amino acid oxidase produced in the cells of the bacteria, and optionally
c) isolating said D-amino acid oxidase.

**18.** Process according to claim 17 wherein the bacteria are strains of the family Enterobacteriaceae.

**19.** A process for oxidative deamination of D-amino acids to keto acids wherein the D-AAO according to claims 7 or 16 is used.

**20.** A process according to claim 19 wherein basic D-amino acids are converted to keto acids.

**21.** A process according to claim 19 wherein racemates of amino acids are purified by deamination of D-amino acids.

**Patentansprüche**

**1.** Isoliertes Polynukleotid, das für ein Protein, umfassend die Aminosäuresequenz gemäß SEQ ID NO: 2 codiert, wobei das Protein D-Aminosäureoxidaseaktivität aufweist.

**2.** Isoliertes Polynukleotid nach Anspruch 1, wobei das Polynukleotid aus Arthrobacter protophormiae isoliert ist.

**3.** Isoliertes Polynukleotid nach den Ansprüchen 1 oder 2 gemäß SEQ ID NO: 1 oder Varianten davon, die durch die Degeneration des genetischen Codes bedingt sind.

**4.** Isoliertes Polynukleotid nach den Ansprüchen 1 oder 2, das aus der SEQ ID NO: 1 oder Fragmenten davon, die für ein Protein bestehend aus der Aminosäuresequenz gemäß SEQ ID NO: 2 codieren, besteht.

**5.** Isoliertes Polynukleotid nach den Ansprüchen 1 oder 2, das das Komplement von SEQ ID NO: 1 umfasst.

**6.** Isoliertes Polynukleotid nach den Ansprüchen 1 oder 2, wobei die Nukleotidsequenz mindestens 90% Identität zu dem Polynukleotid gemäß SEQ ID NO: 1 aufweist.

**7.** Protein, umfassend die Aminosäuresequenz gemäß SEQ ID NO: 2, und wobei das Protein D-Aminosäureoxidaseaktivität aufweist.

**8.** Isoliertes Polynukleotid, bestehend aus mindestens 15 aufeinanderfolgenden Nukleotiden, ausgewählt aus einer von SEQ ID NO: 1 oder einem Komplement von SEQ ID NO: 1 mit der Funktion einer Sonde in einer PCR-Reaktion, die zum Isolieren oder Identifizieren eines Polynukleotids, das für ein Protein, umfassend die Aminosäuresequenz gemäß SEQ ID NO: 2 codiert, eingesetzt wird.

**9.** Isoliertes Polynukleotid nach Anspruch 1, das unter stringenten Bedingungen mit dem Komplement von SEQ ID NO: 1 hybridisiert, wobei die stringenten Bedingungen Waschen mit 0,1 XSSC bei einer Temperatur von 50 bis 68°C umfassen und wobei das Polynukleotid für ein Protein mit D-Aminosäureoxidaseaktivität codiert.

**10.** Expressionskonstrukt, wobei eine DNA-Sequenz nach einem der Ansprüche 1 bis 6 und 9 operativ mit einer Regulationsregion, die fähig ist, die Expression der D-Aminosäureoxidase in einem geeigneten Expressionswirt zu steuern, verbunden ist.

11. Vektor, der fähig ist, eine Wirtszelle zu transformieren, wobei der Vektor ein Expressionskonstrukt nach Anspruch 10 enthält.

12. Vektor nach Anspruch 11, weiterhin **dadurch gekennzeichnet, dass** es sich dabei um ein Plasmid handelt.

13. Transformierte Wirtszelle, umfassend einen Vektor nach Anspruch 10 oder 11.

14. Transformierte Wirtszelle nach Anspruch 13, bei der es sich um ein Bakterium der Familie Enterobacteriaceae handelt.

15. Transformierte Wirtszelle nach Anspruch 13, bei der es sich um ein Bakterium der Gattung Escherichia handelt.

16. Isoliertes Polypeptid, umfassend eine Aminosäuresequenz mit mindestens 90% Identität zu derjenigen von SEQ ID NO: 2 und das D-Aminosäureoxidaseaktivität aufweist.

17. Verfahren zur Herstellung einer D-Aminosäureoxidase, das Folgendes umfasst:

    a) Kultivieren eines rekombinanten Mikroorganismus, umfassend ein überexprimiertes dao-Gen, unter Bedingungen, die der Produktion des dao-Genprodukts förderlich sind; wobei das dao-Gen die Nukleotidsequenz gemäß SEQ ID NO: 1 oder einem der Ansprüche 1 bis 6 und 10 bis 12 umfasst, und
    b) Anreichern der in den Zellen der Bakterien produzierten D-Aminosäureoxidase, sowie gegebenenfalls
    c) Isolieren der D-Aminosäureoxidase.

18. Verfahren nach Anspruch 17, wobei es sich bei den Bakterien um Stämme der Familie Enterobacteriaceae handelt.

19. Verfahren für die oxidative Desaminierung von D-Aminosäuren zu Ketosäuren, wobei die D-AAO nach den Ansprüchen 7 oder 16 eingesetzt wird.

20. Verfahren nach Anspruch 19, wobei basische D-Aminosäuren zu Ketosäuren umgesetzt werden.

21. Verfahren nach Anspruch 19, wobei Racemate von Aminosäuren durch Desaminierung der D-Aminosäuren aufgereinigt werden.

## Revendications

1. Polynucléotide isolé qui code pour une protéine comprenant la séquence d'acides aminés de SEQ ID NO: 2, où ladite protéine a une activité acide aminé D oxydase.

2. Polynucléotide isolé de la revendication 1, dans lequel ledit polynucléotide est isolé à partir d'Arthrobacter protophormiae.

3. Polynucléotide isolé de la revendication 1 ou 2 selon SEQ ID NO: 1 ou des variants de celui-ci qui résultent de la dégénérescence du code génétique.

4. Polynucléotide isolé des revendications 1 ou 2 constitué de SEQ ID N0: 1 ou de fragments de ceux-ci qui codent pour une protéine constituée de la séquence d'acides aminés de SEQ ID NO: 2.

5. Polynucléotide isolé des revendications 1 ou 2 comprenant le complément de SEQ ID NO: 1.

6. Polynucléotide isolé des revendications 1 ou 2, dans lequel ladite séquence nucléotidique est au moins 90 % identique au polynucléotide de SEQ ID NO: 1.

7. Protéine comprenant la séquence d'acides aminés de SEQ ID NO: 2, et dans laquelle ladite protéine a une activité acide aminé D oxydase.

8. Polynucléotide isolé constitué d'au moins 15 nucléotides consécutifs choisi parmi l'un de SEQ ID NO: 1 ou un complément de SEQ ID NO: 1 ayant la fonction d'une sonde dans une réaction de PCR qui est utilisée pour isoler

ou identifier un polynucléotide codant pour une protéine comprenant la séquence d'acides aminés de SEQ ID NO: 2.

9. Polynucléotide isolé de la revendication 1 qui s'hybride dans des conditions stringentes au complément de SEQ ID NO: 1 ; lesdites conditions stringentes comprenant le lavage dans 0,1X SSC à une température de 50 à 68 °C, et le polynucléotide codant pour une protéine ayant une activité acide aminé D oxydase.

10. Construction d'expression dans laquelle une séquence d'ADN de l'une quelconque des revendications 1 à 6 et 9, est fonctionnellement liée à une région de régulation capable de commander l'expression de l'acide aminé D oxydase dans un hôte d'expression adapté.

11. Vecteur capable de transformer une cellule hôte dans laquelle le vecteur contient une construction d'expression selon la revendication 18.

12. Vecteur de la revendication 11, **caractérisé en outre en ce qu'**il est un plasmide.

13. Cellule hôte transformée comprenant un vecteur de la revendication 10 ou 11.

14. Cellule hôte transformée de la revendication 13 qui est une bactérie de la famille Enterobacteriaceae.

15. Cellule hôte transformée de la revendication 13, qui est une bactérie du genre Escherichia.

16. Polypeptide isolé comprenant une séquence d'acides aminés qui est au moins 90 % identique à celle de SEQ ID NO: 2 et a une activité acide aminé D oxydase.

17. Procédé pour la production d'une acide aminé D oxydase comprenant

   a) la culture d'un micro-organisme recombinant comprenant un gène dao surexprimé dans des conditions conductrices pour la production du produit du gène dao ; où ledit gène dao comprend la séquence nucléotidique de SEQ ID NO: 1 ou l'une quelconque des revendications 1 à 6 et 10 à 12 et
   b) l'enrichissement de l'acide aminé D oxydase produite dans les cellules des bactéries, et facultativement
   c) l'isolement de ladite acide aminé D oxydase (D-AAO).

18. Procédé selon la revendication 17, dans lequel les bactéries sont des souches de la famille Enterobacteriaceae.

19. Procédé pour la désamination oxydative d'acides aminés D en céto-acides dans lequel la D-AAO selon les revendications 7 ou 16 est utilisée.

20. Procédé selon la revendication 19 dans lequel des acides aminés D basiques sont convertis en céto-acides.

21. Procédé selon la revendication 19 dans lequel des racémates d'acides aminés sont purifiés par désamination des acides aminés D.

Figure 1

Different stages of purification of D-AAO

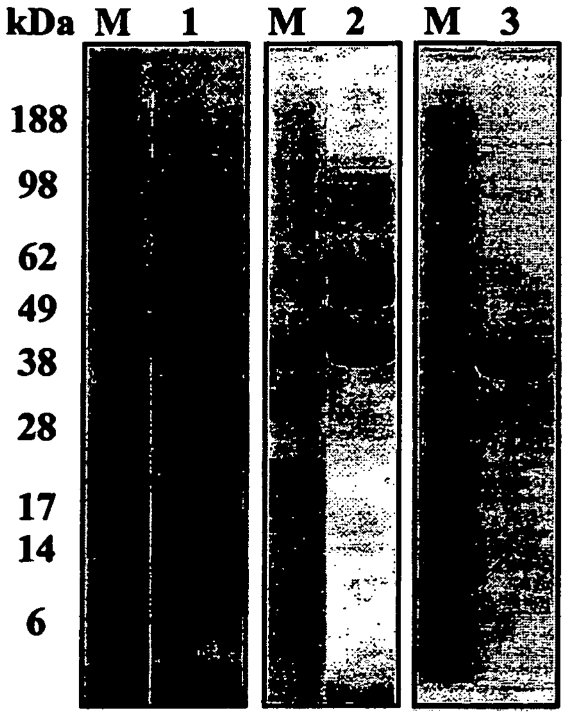

Figure 2: Effect of pH on enzyme activity

Figure 2 a

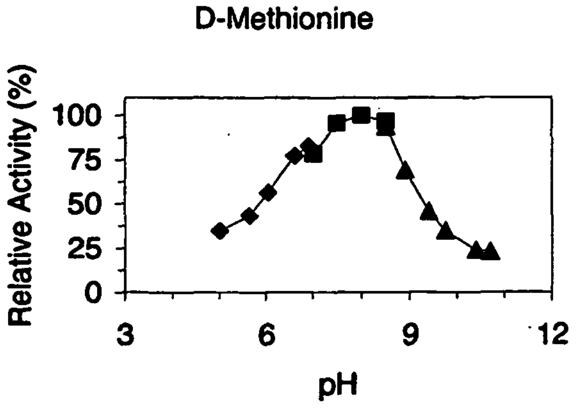


Figure 2 b

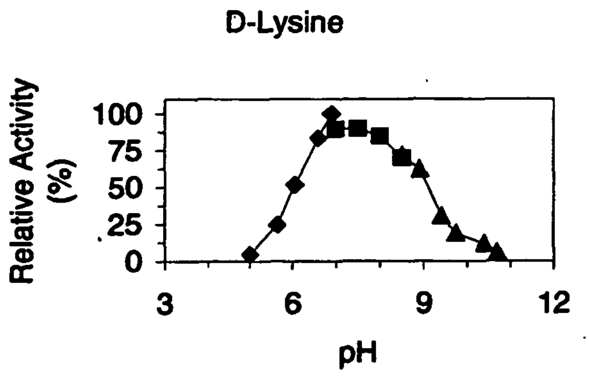


Figure 2 c

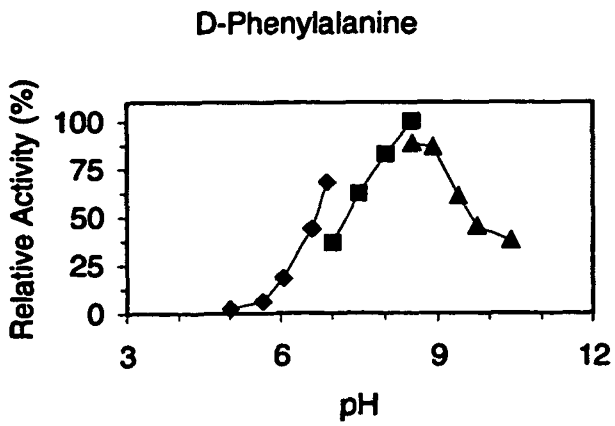

Figure 3: Plasmid pE1D

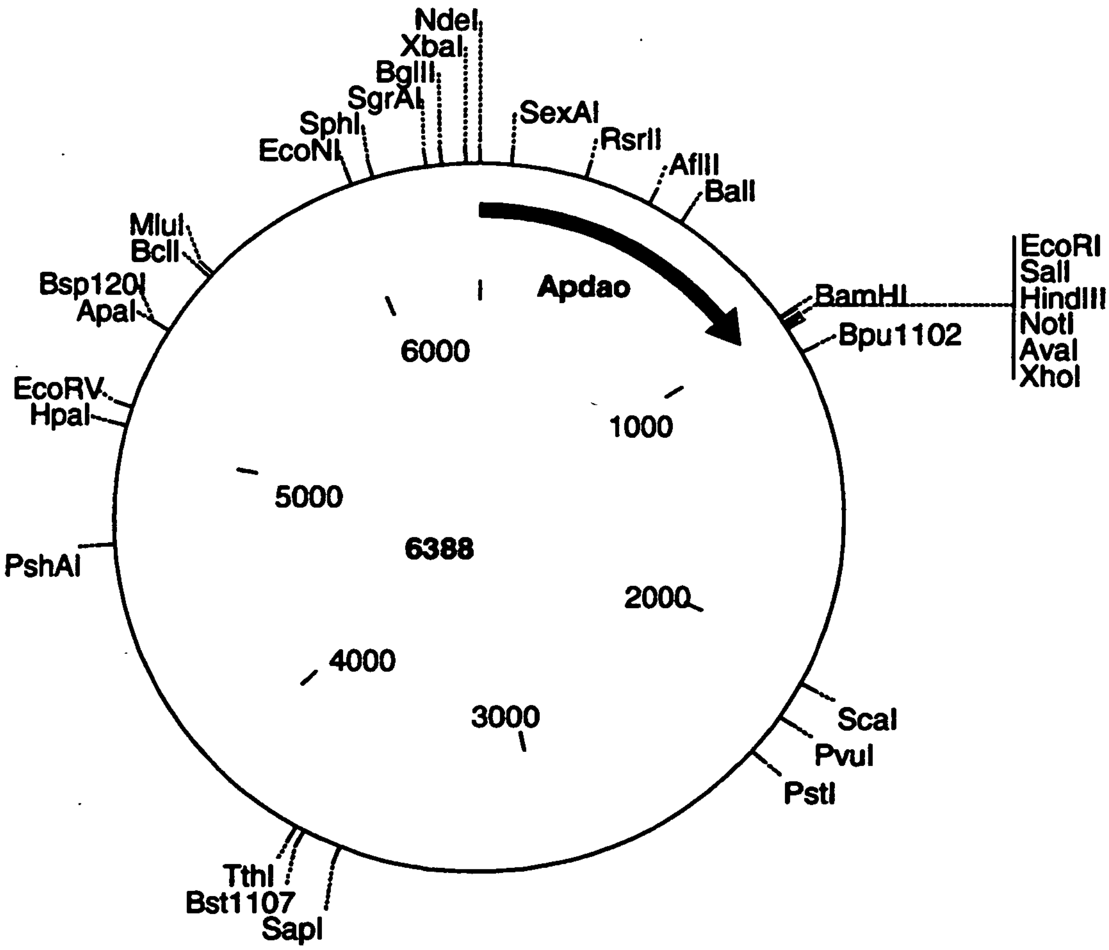

Figure 4: Temperature stability of homogenous D-AAO

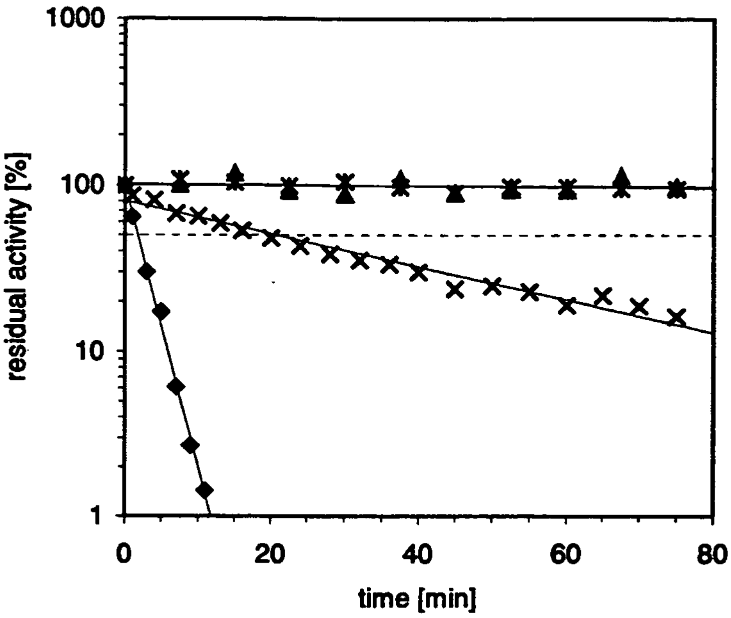

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 19910691
- DE 19903268


### Non-patent literature cited in the description


- **Krebs, H.A.** Metabolism of amino acids. Deamination of amino acids. *Biochem J,* 1985, vol. 29, 77-93
- **Konno, R. et al.** Guinea pig D-amino-acid oxidase cDNA and phylogenetic position. *DNA Seq,* 1999, vol. 10 (2), 85-91
- **Konno, R.** Rat D-amino-acid oxidase cDNA: rat D-amino-acid oxidase as an intermediate form between mouse and other mammalian D-amino-acid oxidases. *Biochim Biophys Acta,* 1998, vol. 1395 (2), 165-70
- **Koibuchi, N. et al.** Localization of D-amino acid oxidase mRNA in the mouse kidney and the effect of testosterone treatment. *Histochem Cell Biol,* 1995, vol. 104 (5), 349-55
- **Konno, R. ; Y. Yasumura.** Activity and substrate specificity of D-amino acid oxidase in kidneys of various animals. *Zool Mag (Tokyo),* 1981, vol. 90, 368-73
- **Pilone, S.M. ; A. Vanoni ; P. Casalin.** Purification and properties of D-amino acid oxidase, an inducible flavoenzyme from Rhodotorula gracilis. *Biochim. Biophys. Acta,* 1987, vol. 914, 136-142
- **Berg, C.P. ; F.A. Rodden.** Purification of D-amino oxidase from Trigonopsis variabilis. *Anal. Biochem.,* 1976, vol. 71 (1), 214-22
- **Gabler, M. ; M. Hensel ; L. Fischer.** Detection and substrate selectivity of new microbial D-amino acid oxidases. *Enzyme Microbial Technol,* 2000, vol. 27 (8), 605-611
- **Sikora, L. ; G.A. Marzluf.** Regulation of L-amino acid oxidase and of D-amino acid oxidase in Neurospora crassa. *Mol. Gen. Genet.,* 1982, vol. 186 (1), 33-9
- **Isogai, T. et al.** Structure and expression of cDNA for D-amino acid oxidase active against cephalosporin C from Fusarium solani. *J. Biochem. (Tokyo),* 1990, vol. 108 (6), 1063-9
- **Fukunaga, S. et al.** Purification and properties of D-glutamte oxidase from Candida boidinii 2201. *J. Ferment. Bioengineer.,* 1998, vol. 85 (6), 579-583
- **Wakayama, M. et al.** Isolation, enzyme production and characterization of D-aspartate oxidase from Fusarium sacchari var. elongatum Y-105. *J. Ferment. Bioengineer.,* 1994, vol. 5 (5), 377-379
- **Justiz, O.H. ; R. Fernandez-Lafuente ; J.M. Guisan.** One-pot chemoenzymatic synthesis of 3'-functionalized cephalosporines (cefazolin) by three consecutive biotransformations in fully aqueous medium. *J. Org. Chem.,* 1997, vol. 62, 9099-9106
- **Diez, B. et al.** Recombinant microorganisms for industrial production of antibiotics. *Biotechnol. Bioeng.,* 1996, vol. 55 (1), 216-226
- **Hanson, R.L. et al.** Enzymatic synthesis of L-6-hydroxynorleucine. *Bioorg. Med. Chem.,* 1999, vol. 7 (10), 2247-52
- **Patel, R.N.** Enzymatic synthesis of chiral intermediates for Omapatrilat, an antihypertensive drug. *Biomol. Eng.,* 2001, vol. 17 (6), 167-82
- **Buto, S. et al.** Evaluation of D-amino acid oxidase from Rhodotorula gracilis for the production of alpha-keto acids: a reactor system. *Biotechnol. Bioeng.,* 1994, vol. 44, 1288-1294
- **Fernandez-Lafuente, R. ; V. Rodriguez ; J.M. Guisan.** The coimmobilization of D-amino acid oxidase and catalase enables the quantitative transformation of D-amino acids (D-phenylalanine) into alpha-keto acids (phenylpyruvic acid). *Enzyme Microbial Technol.,* 1998, vol. 23, 28-33
- **Varadi, M. et al.** Determination of the ratio of D-and L-amino acids in brewing by an immobilised amino acid oxidase enzyme reactor coupled to amperometric detection. *Biosens. Bioelectron.,* 1999, vol. 14 (3), 335-40
- **Sarkar, P. et al.** Screen-printed amperometric biosensors for the rapid measurement of L-and D-amino acids. *The Analyst,* 1999, vol. 124, 865-870
- Winnacker: Gene und Klone. Verlag Chemie, Weinheim, Germany, 1990
- **Sambrook et al.** Molecular Cloning, A Laboratory Manual. Cold Spring Harbor Laboratory Press, 1989
- **Kohara et al.** *Cell,* 1987, vol. 50, 495-508

- **Bathe et al.** *Molecular and General Genetics,* 1996, vol. 252, 255-265
- **Wahl et al.** *Proceedings of the National Academy of Sciences USA,* 1987, vol. 84, 2160-2164
- **Raleigh et al.** *Nucleic Acids Research,* 1988, vol. 16, 1563-1575
- **Börmann et al.** *Molecular Microbiology,* 1992, vol. 6 (3), 317-326
- **Hohn ; Collins.** *Gene,* 1980, vol. 11, 291-298
- **Bolivar.** *Life Sciences,* 1979, vol. 25, 807-818
- **Vieira et al.** *Gene,* 1982, vol. 19, 259-268
- **Grant et al.** *Proceedings of the National Academy of Sciences USA,* 1990, vol. 87, 4645-4649
- **Sanger et al.** *Proceedings of the National Academy of Sciences of the United States of America,* 1977, vol. 74, 5463-5467
- **Sambrook et al.** Molekular Cloning: A Laboratory Manual. Cold Spring Harbor Laboratory Press, 1989
- **Ben-Bassat et al.** *Journal of Bacteriology,* 1987, vol. 169, 751-757
- **O'Regan et al.** *Gene,* 1989, vol. 77, 237-251
- **Sahin-Toth et al.** *Protein Sciences,* 1994, vol. 3, 240-247
- **Hochuli et al.** *Bio/Technology,* 1988, vol. 6, 1321-1325
- **Liebl et al.** *International Journal of Systematic Bacteriology,* 1991, vol. 41, 255-260
- The DIG System User's Guide for Filter Hybridisation. 1995
- **Gait.** Oligonucleotide Synthesis: A Practical Approach. IRL Press, 1984
- **Newton ; Graham.** PCR. Spektrum Akademischer Verlag, 1994
- **Bhavender P. Sharma ; Lorraine F. Bailey ; Ralph A. Messing.** Immobilisierte Biomaterialien - Techniken und Anwendungen. *Angew. Chem.,* 1982, vol. 94, 836-852
- **Dordick et al.** *J. Am. Chem. Soc.,* vol. 194 (116), 5009-5010
- **Okahata et al.** *Tetrahedron Lett.,* 1997, vol. 38, 1971-1974
- **Adlercreutz et al.** *Biocatalysis,* 1992, vol. 6, 291-305
- **Goto et al.** *Biotechnol. Techniques,* 1997, vol. 11, 375-378
- **St Clair et al.** *Angew. Chem. Int Ed Engl.,* January 2000, vol. 39 (2), 380-383
- Textbook of Organic Chemistry. **Beyer-Walter.** Lehrbuch der organischen Chemie. S. Hirzel Verlag, 1991, 822
- **Yu, J., D.-Y. ; Y.-J. Zhang, S. ; Yang, R. Li ; Z.-Y. Yuan.** High expression of Trigonopsis variabilis D-amino oxidase in Pichia pastoris. *J. Mol. Catal. B: Enzymatic,* 2002, vol. 18, 291-7
- **Korz, D. J. ; U. Rinas ; K. Hellmuth ; E. A. Sanders ; W. D. Deckwer.** Simple fed-batch technique for high cell density cultivation of Escherichia coli. *J. Biotechnol.,* 1995, vol. 39, 59-65